(19) Europäisches Patentamt
European Patent Office
Office européen des brevets

(11) EP 0 639 183 B1

(12) **EUROPEAN PATENT SPECIFICATION**

(45) Date of publication and mention of the grant of the patent:
**01.07.1998 Bulletin 1998/27**

(21) Application number: **92905365.0**

(22) Date of filing: **27.02.1992**

(51) Int. Cl.$^6$: **C07D 221/08**, C07D 241/52, A61K 31/435, C07D 401/12

(86) International application number:
**PCT/EP92/00421**

(87) International publication number:
**WO 92/15566 (17.09.1992 Gazette 1992/24)**

# (54) NITROGEN OXIDES OF AZA- AND DIAZA-ANTHRACENEDIONE DERIVATIVES, THEIR PREPARATION AND THEIR USE AS ANTITUMOR AGENTS

STICKSTOFFOXIDE VON AZA- UND DIAZAANTHRACENDIONDERIVATEN, DEREN HERSTELLUNG UND DEREN VERWENDUNG ALS ANTITUMORMITTEL

OXYDES D'AZOTE DE DERIVES D'AZA- ET DIAZA-ANTHRACENEDIONE, LEUR PREPARATION ET LEUR UTILISATION COMME AGENTS ANTITUMORAUX

(84) Designated Contracting States:
**AT BE CH DE DK ES FR GB GR IT LI LU NL SE**

(30) Priority: **08.03.1991 IT MI910618**

(43) Date of publication of application:
**22.02.1995 Bulletin 1995/08**

(73) Proprietor:
**BOEHRINGER MANNHEIM ITALIA S.P.A.**
**20126 Milano (IT)**

(72) Inventors:
- **GANDOLFI, Carmelo, A.**
  **I-20126 Milano (IT)**
- **MENTA, Ernesto**
  **I-20126 Milano (IT)**
- **OLIVA, Ambrogio**
  **I-20126 Milano (IT)**
- **SPINELLI, Silvano**
  **I-20126 Milano (IT)**
- **TOGNELLA, Sergio**
  **I-20126 Milano (IT)**

(74) Representative:
**Weber, Manfred, Dr. et al**
**c/o Boehringer Mannheim GmbH,**
**Patentabteilung,**
**Sandhoferstrasse 116**
**68298 Mannheim (DE)**

(56) References cited:
- **JOURNAL OF MEDICINAL CHEMISTRY. vol. 28, no. 8, 1985, WASHINGTON US pages 1124 - 1126; A.P. KRAPCHO: 'Synthesis and antineoplastic evaluations of 5,8-bis[(aminoalkyl)amino]-1-azaa nthracene-9,10-diones' cited in the application**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.3/3.4

## Description

This invention is directed to nitrogen oxides of aza and diaza anthracenediones, and more particularly, to 6,9-substituents which are (aminoalkyl)amino substituents. These compounds have been shown to have antitumor activity in vitro and in vivo.

Certain 1,4-bis[(aminoalkyl)amino]anthracene-9,10-drones have been reported which show antitumor activity in clinical trials. Of particular interest has been ametantrone, 1,4-bis{[2-(2-hydroxyethylamino)ethyl]amino]anthracene-9,10-dione and mitoxantrone, 5,8-dihydroxy-1,4-bis([2-(2-hydroxyethylamino)ethyl]amino]anthracene-9,10-dione. (Zee-Cheng et al., J. Med. Chem., (1978), 21, 291-4; Cheng et al., "Progress in Medicinal Chemistry", Ellis, G.P. and West, G.B., eds.; Elsevier: Amsterdam, 1983; pp. 20, 83 and references cited therein). Mitoxantrone is a broad spectrum oncolytic agent, whose activity is similar to that of the anthracycline antibiotic doxorubicin. Clinical trials have demonstrated that mitoxantrone has particularly promising activity in the treatment of advanced breast cancer, acute leukemia and lymphoma (Legha, Drugs of Today, (1984), 20, 629). Although animal studies have demonstrated a diminished cardiotoxicity in comparison to doxorubicin, some clinical cardiotoxicity has been observed also with mitoxantrone, mostly in patients previously treated with doxorubicin (R. Stuart Harris et al., Lancet, (1984), 219, and references cited therein).

Ametantrone has been reported to be, in animals, about 10-fold less potent and cardiotoxic than mitoxantrone. Because a delayed toxicity is observed only with mitoxantrone after administration of the two drugs by the i.p. route to non-tumor bearing rats at equieffective antitumor dosages, it is suggested that the presence of the 5,8-dihydroxy substitution in mitoxantrone might be implicated in the delayed deaths (Corbet et al., Cancer Chemother. Pharmacol., (1981), 6, 161).

In addition, both mitoxantrone and ametantrone have a remarkable myelodepressive toxicity and both compounds show cross-resistance to cell hystotypes developing resistance against doxorubicin mediated by overexpression of glycoprotein P. Such a resistance, which is named multidrug resistance, involves a number of antitumor antibiotics, among which amsacrine and podophyllotoxinic derivatives, and it is one of the main reasons for therapeutical failures in the treatment of soled tumors with said antibiotics.

Therefore, search is required for novel anthracenedione antitumor agents, having a higher therapeutical index than mitoxantrone and being effective both in inhibiting or delaying the growth of those solid tumors which are more refractory to chemotherapeutic treatment (such as lung, breast and colon tumors) and against tumor histotypes developing multidrug resistance.

In the search for safer, active analogues of anthracenediones, compounds bearing hydroxysubstituents and/or (aminoalkyl)amino side chains at various positions on the anthracene-9,10-dione nucleus have been studied (C.C. Cheng et al., Drugs of the Future, (1983), 8, 229) without notable improvement.

Aza- and diaza anthracene-9,10-diones such as 6,9-bis(ethoxycarbonylamino)benzo[g]quinoline-5,10-dione (1), 6,9-bis(ethoxycarbonylamino)benzo[g]isoquinoline-5,10-dione (2), 6,9-bis(ethoxycarbonylamino)benzo[g]-quinazoline-5,10-dione (3), were disclosed by Potts et al. (Synthesis, (1983, 31). These compounds were reported to be related to the antitumor 1,4-bis[(aminoalkyl)amino]anthracene-9,10-diones; however no antitumor activity data was reported for any of the above compounds. 1-[(aminoalkyl)amino] derivatives (4) of 6,9-dihydroxybenzo[g]isoquinoline-5,10-dione related to mitoxantrone have been disclosed as DNA intercalators but they were completely devoid of any "in vitro" or "in vivo" antitumor activity (Croisy-Delcey et al., Eur. J. Med. Chem., (1988), 23, 101-106).

The 6,9-bis[(aminoalkyl)amino]benzo[g]quinoline-5,10-diones of formulas 5a-d (see Table I) were disclosed in J. Med. Chem. (1985), 28, 1124-26, where they were referred to as 5,8-bis[(aminoakyl)amino]-1-azaanthracenediones.

As shown in table II hereinbelow reported the prior art compounds 5 are clearly less active than the corresponding carbocyclic analogues (6).

In fact, as reported in table II, the compound 5a and 5b are less cytotoxic than the analogues 6a and 6b. Moreover the compound 5a, which is poorly cytotoxic in vitro, is inactive in vivo, and it is both less active and less potent than the carbocyclic analogue 6a.

Even though the introduction of an heteroatom is a common process in the medicinal chemistry, its effect must be evaluated case by case.

In this particular case of aza-analogues of anthracenediones the prior art teachings clearly show that the introduction of a nitrogen atom into the anthracenedione skeleton is detrimental for antitumor activity. In fact the aza-substituted anthracenediones are less cytotoxic than the corresponding anthracenediones and inactive "in vivo".

Thus a person skilled in the art, would have not considered the introduction of heteroatoms into the anthracenedione skeleton as a possible way to obtain more effective antitumor agents.

It is well known that the screening for the disco-very of the antitumor agent mitoxantrone (J. Med. Chem., (1978), 21, 291-4) among a number of analogues has been carried out by using the experimental model of murine leukemia P388: this model is then predictive of antitumor activity in humans at least for this class of antitumor antibiotics. Many other clinically active antitumor antibiotics are active on murine leukemias P388 and L1210 such as m-amsacrine and doxorubicin.

Moreover mitoxantrone has shown good activity in other significative experimental tumors such as murine Lewis lung carcinoma and MXI human mammary carcinoma.

We have now discovered that nitrogen oxides of aza and diaza anthracene-9,10-diones are active as antitumor agents: they are highly effective against murine leukemias L1210 and P388 and against Lewis lung carcinoma and MXI human mammary carcinoma.

**Table I**

Prior art compounds

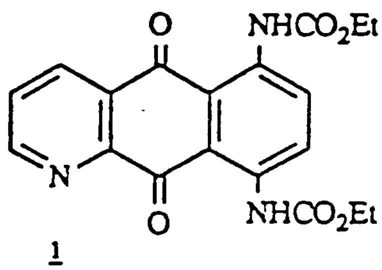

1

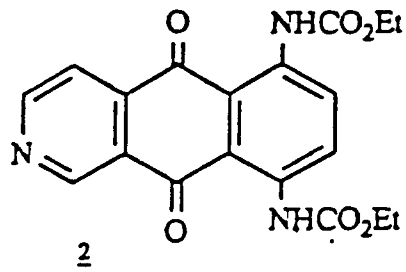

2

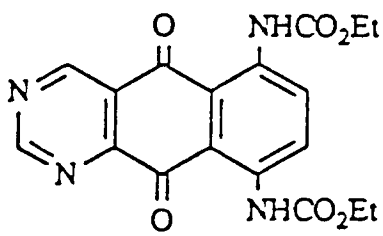

3

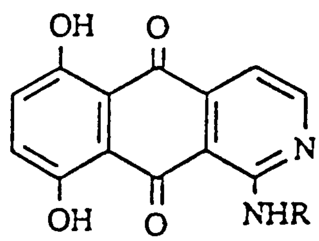

4a R= $CH_2CH_2N(CH_3)_2$

4b R= $CH_2CH_2CH_2N(Et)_2$

4c R= $CH_2CH_2NHCH_2CH_2OH$

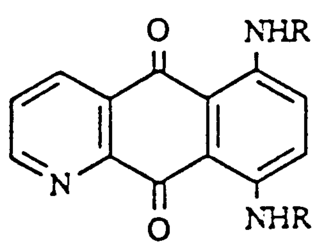

5a R= $CH_2CH_2N(CH_3)_2$

5b R= $CH_2CH_2N(Et)_2$

5c R= $CH_2CH_2CH_2N(CH_3)_2$

5d R= $CH_2CH_2NHCOCH_3$

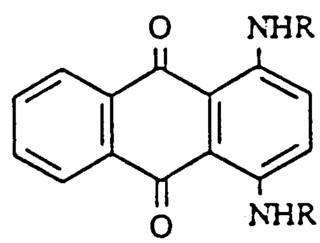

6a R= $CH_2CH_2N(CH_3)_2$

6b R= $CH_2CH_2N(Et)_2$

Table II

| Antitumor activity of prior art compounds 5 and 6 against murine L 1210 leukemia (from J. Med. Chem. (1985), 28, 1124-1126) | | | |
|---|---|---|---|
| | in vitro $ID_{50}$ (μg/ml) | in vivo | |
| | | Dose (mg/kg) | T/C |
| 5a | 0.16 | 50 | 130 |
| 5b | 2.4 | 100 | toxic |
| 5c | 1.7 | | |
| 6a | 0.08 | 30 | 150 |
| 6b | 0.30 | | |

The compounds of the invention have the following formula (I)

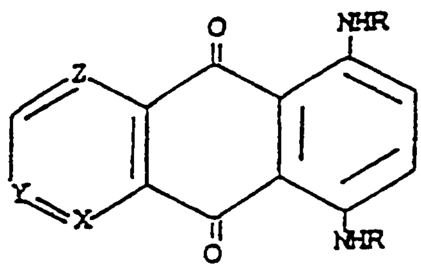

(I)

wherein

X, Y and Z are CH, N or N → O thus forming an azine or diazine ring, with the proviso that:

- at least one of X, Y and Z is N → O;

R is a substituted ($C_2$-$C_{10}$)-alkyl group, selected from the group consisting of :

- residues of formula $-(CH_2)_p-OH$, wherein p is an integer from 2 to 4;
- residues of formula $-(CH_2)_p-NH_2$ wherein p is as defined above;
- residues of formula $-(CH_2)_p-NR^2R^3$ wherein p is as defined above and $R^2$ and $R^3$ are ($C_1$-$C_6$)-alkyl groups or, taken together with the nitrogen atom, they form an aziridine, pyrrolidine, morpholine, piperazine, N-methylpiperazine, N-benzylpiperazine or piperidine ring;
- residues of formula

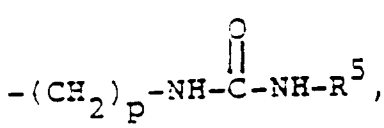

  wherein p is as defined above and $R^5$ is ($C_1$-$C_6$)-alkyl or phenyl;
- residues of formula $-(CH_2)_p-NH-(CH_2)_q-OH$ wherein p and q are independently an integer 2 to 4;
- residues of formula

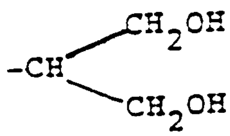

or

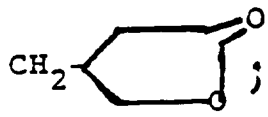

;

and the pharmaceutically acceptable salts thereof.

The present invention also relates to the tautomeric forms, the racemic and diastereoisomeric mixtures of the compounds of formula (I) as well as the single enantiomers and diastereoisomers thereof.

Examples of pharmaceutically acceptable acids which can salify compounds (I) are inorganic acids, such as hydrochloric, hydrobromic, sulfuric, phosphoric, pyrophosphorc acids or organic acids, such as acetic, propionic, citric, benzoic, lactic, maleic, malic, fumaric, succinic, tartaric, glutamic, aspartic, gluconic, ascorbic acids or other conventionally used acids.

Specific examples of azine and diazine rings containing at least one nitrogen-oxide function are those represented by the following formulae:

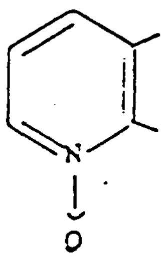

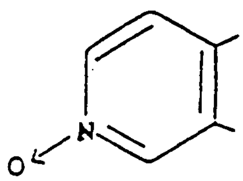

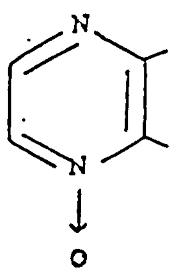

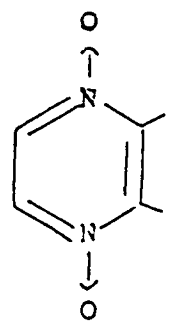

In compounds (I), the word "phenyl" means phenyl rings which can optionally contain substituents such as ($C_1$-$C_4$)-alkyl groups; $CF_3$; halogen atoms; nitro, amino, acetylamino, formylamino, dimethylamino, diethylamno, hydroxy, methoxy and ethoxy groups.

Preferred examples of ($C_1$-$C_{10}$)-alkyl groups are methyl, ethyl, n-propyl, sec-propyl, n-butyl, sec-butyl, tert-butyl, n-hexyl and n-pentyl.

Preferred are those compounds of formula (I) in which R is a substituted ($C_2$-$C_{10}$)-alkyl group, selected from the group consisting of :

- residues of formula $-(CH_2)_p-OH$, wherein p is the integer 2, 3 or 4;
- residues of formula $-(CH_2)_p-NH_2$ wherein p is as defined above;
- residues of formula $-(CH_2)_p-NR^2R^3$ wherein p is as defined above and $R^2$ and $R^3$ are ($C_1$-$C_6$)-alkyl groups or, taken together with the nitrogen atom, they form an 1-aziridine, 1-pyrrolidine, 4-morpholine, 1-piperazine, 4-methyl-1-piperazine, 4-benzyl-1-piperazine or 1-piperidine ring;
- residues of formula

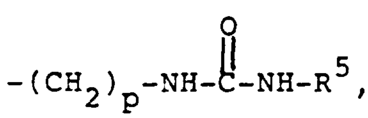

wherein p is as defined above and $R^5$ is ($C_1$-$C_6$)-alkyl or phenyl;
- residues of formula $-(CH_2)_p-NH-(CH_2)_q-OH$ wherein p and q are independently an integer selected from the group of 2,3 or 4;
- residues of formula

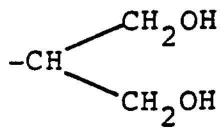

or

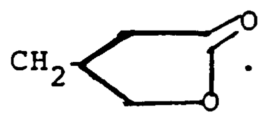

Specific examples of preferred compounds of the invention are the following ones:

6,9-bis[N-(2-aminoethyl)amino]benzo[g]isoquinoline-5,10-dione-2-oxide;
6,9-bis[N-(2-dimethylaminoethyl)amino]benzo[g]isoquinoline-5,10-dione-2-oxide;
6,9-bis(N-[2-(2-hydroxyethylamino)ethyl]amino]benzo[g]isoquinoline-5,10-dione-2-oxide;
6,9-bis(N-[2-(4-morpholinyl)ethyl]amino}benzo[g]isoquinoline-5,10-dione-2-oxide;
6,9-bis[N-(2-diethylaminoethyl)amino]benzo[g]isoquinoline-5,10-dione-2-oxide;
6,9-bis{N-[2-(1-pyrrolidinyl)ethyl]amino}benzo[g]isoquinoline-5,10-dione-2-oxide;
6,9-bis[N-(3-hydroxypropyl)amino]benzo[g]isoquinoline-5,10-dione-2-oxide;
6,9-bis[N-(1,1-bis(hydroxymethyl)methyl)amino]benzo[g]isoquinoline-5,10-dione-2-oxide;
6,9-bis{N-[2-(1-aziridinyl)ethyl]amino}benzo[g]isoquinoline-5,10-dione-2-oxide;
6,9-bis[N-(3-aminopropyl)amino]benzo[g]isoquinoline-5,10-dione-2-oxide;
6,9-bis[N-(4-aminobutyl)amino]benzo[g]isoquinoline-5,10-dione-2-oxide;
6,9-bis{N-[2-(methylcarbamoylamino)ethyl]amino}benzo[g]isoquinoline-5,10-dione-2-oxide;
6,9-bis{N-[2-(phenylcarbamoylamino)ethyl]amino}benzo[g]isoquinoline-5,10-dione-2-oxide;
6,9-bis[N-(2-aminoethyl)amino]benzo[g]quinoline-5,10-dione-1-oxide;
6,9-bis[N-(2-dimethylaminoethyl)amino]benzo[g]quinoline-5,10-dione-1-oxide;
6,9-bis{N-[2-(2-hydroxyethylamino)ethyl]amino}benzo[g]quinoline-5,10-dione-1-oxide;
6,9-bis[N-(2-aminoethyl)amino]benzo[g]quinoxaline-5,10-dione-1-oxide;
6,9-bis[N-(2-aminoethyl)amino]benzo[g]quinoxaline-5,10-dione-1,4-dioxide;
6,9-bis[N-(2-dimethylaminoethyl)amino]benzo[g]quinoxaline-5,10-dione-1-oxide;
6,9-bis[N-(2-dimethylaminoethyl)amino]benzo[g]quinoxaline-5,10-dione-1,4-dioxide;
6,9-bis{N-[2-(2-hydroxyethylamino)ethyl]amino}benzo[g]quinoxaline-5,10-dione-1-oxide;
6,9-bis{N-[2-(2-hydroxyethylamino)ethyl]amino}benzo[g]quinoxaline-5,10-dione-1,4-dioxide.

The compounds of the invention of formula (I) can be prepared by reacting a compound of formula (II)

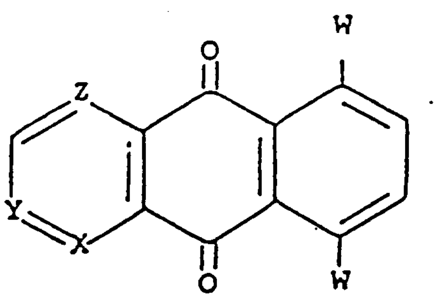

(II)

wherein

X, Y and Z are as defined above;

W is a fluorine atom or a

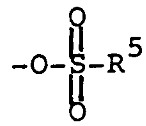

group, $R^5$ being as defined above;

with a compound of formula (III)

$$H_2N\text{-}R_a \qquad \text{(III)}$$

wherein $R_a$ has the same meanings as defined in formula (I) for R or it is a group which may be converted to R, to give a compound of formula (Ia):

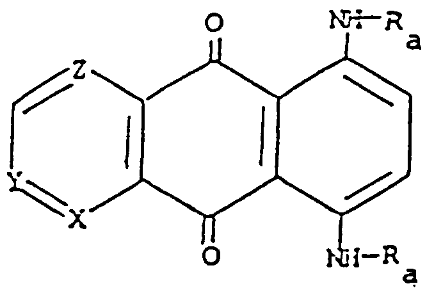

(Ia)

and then optionally performing one or more of the following steps:

a) when $R_a$ is other than R, converting $R_a$ into R to obtain a compound of formula (I);
b) when $R_a$ is one of the groups defined above for R in compounds of formula (I), and in which case the compounds of formula (Ia) are the same as the compounds of formula (I), $R_a$ can be optionally converted into another $R_a$ group to give another compound of formula (I);
c) optional salification and/or solvation of the obtained compounds of formula (I) or separation of the isomers thereof.

The reaction of compound (II) with a compound of formula (III) is generally carried out in the presence of a stoichiometric amount or a slight molar excess of compound (III) in a solvent such as methylene chloride, chloroform, 1,1,1-trichloroethane, dimethoxyethane, tetrahydrofuran, dimethylformamide, N,N,N',N'-tetramethylethylenediamine, pyridine, picoline, quinoline and mixtures thereof, or, if desired, using compound (III) itself as the solvent, optionally in the presence of an inorganic base such as an alkali or alkaline-earth carbonate or hydrogen carbonate or an organic base such as triethylamine or other tertiary amines, at a temperature from 0°C to the reflux temperature of the solvent.

Preferably the reaction is carried out in pyridine at 50°C, using from 2 to 5 equivalents of compound (III) per equivalent of compound (II).

The optional removal of primary- or secondary-amine protecting groups or hydroxy-protecting groups optionally present in compounds of formula (Ia) can be carried out according to well-known methods, such as hydrolysis of the esters and amides of compounds of formula (Ia) with organic or inorganic acids or bases, or selective cleavage of the protecting groups, using suitable reducing and oxidizing agents, the preferred reagent of course depending on the nature of the protecting group.

Compounds of formula (II) can be prepared by oxidizing a compound of formula (IV)

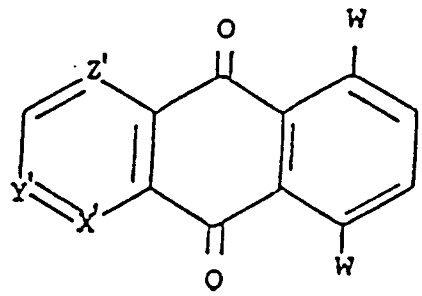

(IV)

wherein

X', Y' and Z' have the same meanings as X, Y and Z, with the exclusion of N → O, so as to form an azine or diazine ring;
W is as defined above;

with oxidizing agents such as peracids, peroxides or salts thereof.

The oxidation of compounds (IV) to give compounds of formula (II) is generally carried out using a stoichiometric amount or a slight excess of an oxidizing agent, in a solvent such as chloroform, methylene chloride, 1,2-dichloroethane, acetic acid, optionally in the presence or in the absence of an alkali or alkaline-earth metal carbonate or bicarbonate, at a temperature from 0°C to the reflux temperature of the solvent and for a time ranging from a few hours to some days, depending on the experimental conditions.

Examples of the oxidizing agents which can effectively be used to oxidize compounds of formula (IV) to compounds of formula (II) include hydrogen peroxide; peracetic, trifluoroperacetic, perbenzoic and m-chloroperbenzoic acids; monoperoxyphthalic acid or the magnesium salt thereof; persuccinic acid. The reaction is preferably effected using at least 1,2 equivalents of an organic peracid, such as m-chloroperbenzoic acid, per nitrogen-oxide function which is desired, in a solvent such as methylene chloride.

Compounds (IV) can be prepared by means of a multi-step process, including a Friedel-Crafts condensation between a compound of formula (V)

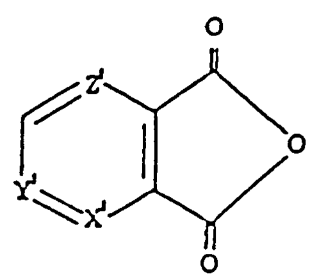

(V)

wherein X', Y' and Z' are as defined above;

with para-difluorobenzene, which results in compounds having the formulae (VI) and (VIa)

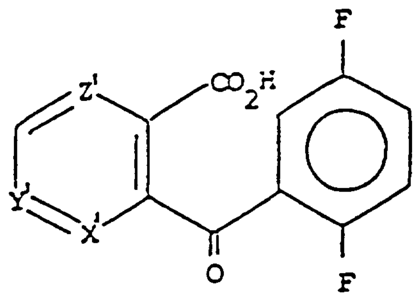

(VI)

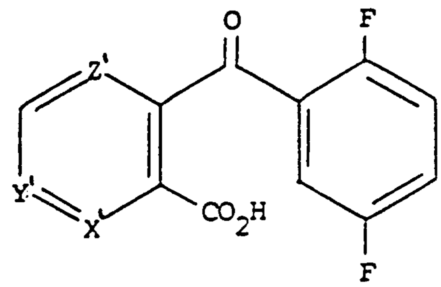

(VIa)

which are subsequently subjected to a cyclization reaction to yield a compound of formula (IV) wherein W is fluorine. Alternatively, Friedel-Crafts bis-acylation of a 1,4-dialkoxybenzene with a compound of formula (V) gives a compound of formula (VII)

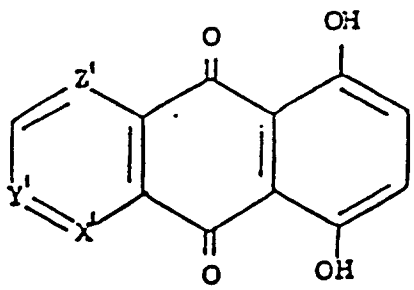

(VII)

wherein X', Y' and Z' are as defined above;

which is subsequently reacted with a compound of formula (VIII)

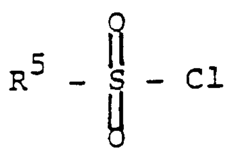

(VIII)

wherein $R^5$ is as defined above,

to give a compound of formula (IV) wherein W is

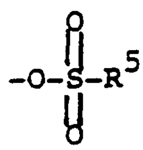

The reaction of a compound of formula (V) with para-difluorobenzene, to give the compounds of formula (VI) and

(VIa), is generally carried out in the presence of at least two equivalents of a Lewis acid, such as $AlCl_3$, $AlBr_3$, $ZnCl_2$ and the like. The reaction is performed preferably using para-difluorobenzene as the solvent, in the presence of an amount of aluminium trichloride which can range from 2.5 to 4.0 moles per mole of compound (V) at 90°C for some hours.

Cyclization of the compounds of formulae (VI) and (VIa) to gave a compound of formula (IV) in which W is fluorine, can be carried out using condensing agents such as polyphosphoric acid, PPE, sulfuric acid, or oleum , at temperature from room temperature to 180°C. Preferably, cyclization is effected using 20% $SO_3$ oleum at 140°C for about 10 hours.

Bis-acylation of a 1,4-dialkoxybenzene with a compound of formula (V) is preferably carried out using 1,4-dimethoxybenzene, in an eutectic mixture of aluminium chloride and sodium chloride at 180°C, and it generally is completed in a few minutes.

The reaction of a compound of formula (VII) with a compound of formula (VIII) to give a compound of formula (IV) in which W is

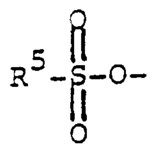

is effected according to conventional methods.

Usually the reaction is carried out in the presence of a stoichiometric amount or a slight excess of a compound of formula (VIII) in a solvent, such as methylene chloride, chloroform, dimethoxyethane, tetrahydrofuran, dimethylformamide, pyridine or mixtures thereof, in the optional presence of an organic or inorganic base, or according to Schotten-Baumann method, at a temperature from 0°C to the reflux temperature of the solvent.

Generally the reaction is carried out in pyridine at room temperature and it is complete after 48 hours.

Compounds of formula (V) can be prepared from compounds of formula (IX)

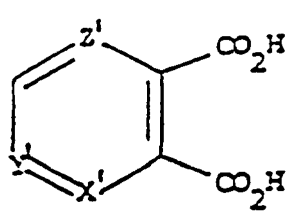

(IX)

wherein X', Y' and Z' are as defined above, by reaction with an appropriate dehydrating agent, such as carboxylic acid anhydrides (e.g. acetic or trifluoroacetic anhydrides), carbodiimides, such as N,N'-dicyclohexylcarbodiimide, methoxyacetylene, or phosphoric anhydride.

Acetic anhydride at a temperature from 80°C to the solvent's reflux temperature, or N,N'-dicyclohexylcarbodiimide in an aprotic solvent at a temperature from 20°C to the solvent's reflux temperature, are preferred reaction conditions.

Compounds (III) and (IX) are known or commercially available. Useful teachings to prepare some compounds of formula (III) can be found in J. Org. Chem. (1959), 24, 818-820; J. Med. Chem. (1980), 33, 112-117; J. Med. Chem., (1981), 34, 184-189; Synthetic Comm., (1990), 20(16), 2559-2564.

The evaluation of the biological activity for the compounds of this invention was performed "in vitro" and "in vivo" following the protocols developed by the U.S. National Cancer Institute.

The evaluation of the "in vitro" cytotoxic activity of the compounds of the invention was performed using a human colon adenocarcinoma cell line (Lovo) isolated from a metastatic nodule and a subline with aquired resistance to a number of antitumor agents, among which doxorubicin, VP-16 and vincristine. ThiS subline (named Lovo/DX) shows reduced accumulation of doxorubicin and overexpression of a protein (Grandi, M., Geroni, C., Giuliani, F.C., British J. Cancer, (1986), 54, 515). The compounds were tested according to the MTT assay (Mosman, T. "Rapid Colorimetric assay for cellular growth and survival: application to proliferation and cytotoxicity assay", J. Immunol. Methods, (1983), 65, 55-63; Green. L.M., "Rapid Colorimetric assay for cell viability; application to the quantitation of cytotoxic and growth inhibitory lymphokines", J. Immunol. Methods, (1984), 70, 257-268) in comparison with ametantrone and doxorubicin.

In general, representative compounds of this invention were equally cytotoxic as ametantrone in the Lovo cell line. For example a representative compound of the invention such as 6,9-bis[N-(2-dimethylaminoethyl)amino]benzo[g]isoquinoline-5,10-dione-2-oxide, described in example 12 of the experimental section, showed an $IC_{50}$ of 0.44 μg/ml (144 hours drug-exposure) while ametantrone in the same experimental conditions showed an $IC_{50}$ of 0.33 ± 0.1 μg/ml.

When ametantrone was tested in the Lovo/DX cell line, a resistance index RI (defined as the ratio of the $IC_{50}$ for the resistant cell line to the $IC_{50}$ for the sensitive cell line) as high as 101.2 was found, showing that this subline did have an acquired resistance to ametantrone. On the other hand a representative compound of the invention such as 6,9-bis[N-(2-dimethylaminoethyl)amino]benzo[g]isoquinoline-5,10-dione-2-oxide (7a) (see table III for structures) showed on this resistant subline an $IC_{50}$ of 2.68 μg/ml with an R.I. of 6.1 while doxorubicin showed on this resistant cell lane an $IC_{50}$ of 4.28 ± 1.8 μg/ml and R.I. = 142.7.

These in vitro data suggest that representative compounds of this invention may be useful in order to overcome the multidrug resistance mediated mechanism of tumor resistance.

Studies of the biological activity "in vivo" of representative compounds of the invention were performed using the P 388 and L 1210 murine leukemia models.

P 388 Murine leukemia cells were intravenously (iv) injected in CD2FI mice. Treatment was initiated approximately 24 hours after tumor transplantation and dosages of the drug were administered iv according to preestablished protocols, usually at 3-day intervals. The studies were done over a 60 day period and the date of death for each animal was recorded. The % T/C was determined using the median survival time (MST) for each group according to the formula

$$\% \ T/C = [(\text{MST treated})/(\text{MST control})] \times 100.$$

For example a representative compound of this invention, namely 6,9-bis{[2-(amino)ethyl]amino]benzo[g]isoquinoline-5,10-dione-2-oxide as the dimaleate salt described in example 13 showed an activity superior to that of mitoxantrone. Moreover this representative compound of the invention showed antileukemic activity over a wide range of well tolerated dosages and in particular it was active at dosages which were lower than the maximum tolerated dose, providing indication for a more favourable therapeutic index in comparison to mitoxantrone. The results are shown in table (IV)

**Table III**

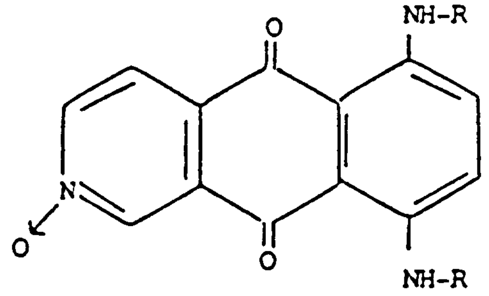

7a R = $CH_2CH_2N(CH_3)_2$

7b R = $CH_2CH_2NH_2$ (dimaleate salt)

Table IV

| ANTITUMOR ACTIVITY OF THE REPRESENTATIVE COMPOUND OF THE INVENTION 6,9-BIS[N(2-AMINOETHYL)AMINO]BENZO[G]ISOQUINOLINE-5,10-DIONE-2-OXIDE DIMALEATE (<u>7B</u>) AND MITOXANTRONE (MITOX) AGAINST P 388 MURINE LEUKEMIA (iv/iv 1,4,7)[1] | | | |
|---|---|---|---|
| Compound[2)] | Dose (mg/kg/day) | T/C%[3)] (range) | TOX[4)] |
| <u>7b</u> | 18 | 122 | 0/8 |
| | 27 | 133 | 0/8 |
| | 40 | 144,166,194 | 1/24 |
| | 60 | 200,225 | 0/17 |
| | 80 | 211,262 | 4/17 |
| MITOX | 2 | 171(154-212) | 0/50 |
| | 3 | 185(167-217) | 12/84 |
| | 4 | 118(89-133) | 20/24 |

1) CD2FI mice were transplanted iv with $10^6$ cells/mouse. Treatment was given iv on days 1,4,7 after tumor transplantation (day 0).
2) Compound <u>7b</u> and MITOX were dissolved in sterile water.
3) Median survival time of treated mice/median survival time of controls x 100.
4) Number of toxic deaths/total number of mice.

The antitumor activity of representative compounds of this invention was evaluated also in the L 1210 murine leukemia model.

L 1210 leukemia cells were intravenously (iv) injected in CD2FI mice and treatment was initiated approximately 24 hours after tumor transplantation. Dosages of the drugs were administered iv according to preestablished protocols, usually at 3-day intervals. The studies were done over a 60-day period and the date of death for each animal was recorded. The % T/C was determined using the median survival time (MST) for each group according to the formula

$$\% \ T/C = [(MST\ treated)/(MST\ control)] \times 100.$$

The results are shown in table (V) from which the superior antileukemic activity of the representative compound of the invention 6,9-bis[N-(2-aminoethyl)amino]benzo[g]isoquinoline-5,10-dione-2-oxide dimaleate (<u>7b</u>) of example 13 in comparison to mitoxantrone is evident.

Table V

| ANTITUMOR ACTIVITY OF THE REPRESENTATIVE COMPOUND OF THE INVENTION 6,9-BIS[N-(2-AMINOETHYL)AMINO]BENZO[G]ISOQUINOLINE-5,10-DIONE-2-OXIDE DIMALEATE (<u>7B</u>) AND MITOXANTRONE (MITOX) AGAINST MURINE LEUKEMIA L1210 (iv/iv 1,4,7)[1] | | | | |
|---|---|---|---|---|
| Compound[2)] | Dose (mg/kg/day) | T/C%[3)] (range) | TOX[4)] | LTS[5)] |
| <u>7b</u> | 40 | 266 | 0/8 | 0/8 |
| | 60 | 333 | 0/10 | 0/10 |
| MITOX | 2 | 208 | 0/8 | 0/8 |
| | 3 | 216 | 0/8 | 0/8 |

1) CD2FI mice were transplanted iv with $10^5$ cells/mouse. Treatment was given iv on days 1,4,7 after tumor transplantation (day 0).
2) Compound <u>7b</u> and MITOX were dissolved in sterile water.
3) Median survival time of treated mice/median survival time of controls x 100.
4) Number of toxic deaths/total number of mice.

Representative compounds of the invention, when administered to test animals bearing experimental tumors such as P388/DOXO doxorubicin-resistant murine leukemia P388, Lewis lung carcinoma, human mammary carcinoma MXI, LOVO human colorectal tumor, LOVO/DOXO doxorubicin-resistant human colorectal tumor, induce a statistically significant regression in the experimental tumors compared to the control animals treated with the only vehicle. The activity of the induced regression is directly proportional to the used dosage (pharmacological dose-related effect) and to the dosage scheme (e.g. single administration in the day after tumor transplantation or repeated administrations at days 3, 5, 9 or 3, 5, 9, 14 after the tumor implantation). The compounds of the invention, when administered at their maximum tolerated doses, induce a higher regression of these experimental tumors than that caused by doxorubicin and mitoxantrone, at their maximum tolerated doses, under the same experimental conditions. Particularly, the compounds of the invention are markedly more effective than the control drugs in inhibiting the growth of solid tumors which are doxorubicin- and mitoxantrone-resistant.

Therefore, the available test data prove that the compounds of the invention have the following features:

- they have a higher therapeutic index than mitoxantrone, since the range of therapeutically effective tolerated doses in the animal is markedly wider than that of mitoxantrone;
- they are effective in inhibiting growth of liquid and solid experimental tumors, such as murine leukemias, murine lung solid tumors, human mammary and colon solid tumors which are predictive of good results in human;
- they are effective against experimental solid tumors which are doxorubicin- and mitoxantrone-resistant (multidrug-resistant).

Therefore, due to these characteristics, the compounds of the invention are expected to be operative against human leukemias and solid tumors and they can be used as the active ingredients in therapeutical compositions for the regression and/or the treatment of tumors in mammals, when administered in amounts ranging from 0.001 g to about 0.4 g per kg body weight per day. A preferred dosage regimen for optimum results would be from about 0.001 g to about 0.05 g per kg body weight per day, using unitary dosages so as to administer from about 0.07 g to about 3.5 g of active compound to a subject of about 70 kg body weight, in a 24 hour period. This dosage regimen can be adjusted to provide optimum therapeutical response. For example, several divided doses can be administered according to the requirements of the therapeutical situation. A remarkable practical advantage is that the active compound can be administered by the oral, intravenous, intramuscular or subcutaneous routes.

The compositions of the present invention containing compounds of formula (I) can be used for the treatment of blood cancer diseases such as leukemia or other solid and non-solid tumors such as lung carcinoma, colon carcinoma, melanocarcinoma and mammary tumors. As used herein, by regression or treatment is meant arresting or delaying the growth of the tumor. Suitable administration forms for intravenous injection are physiological sterile solutions. For the intramuscular or intraperitoneal administrations, oily or aqueous injectable preparations are also suitable, whereas suitable forms for the oral administrations are liquid or solid preparations such as syrups, capsules or tablets.

The invention is further illustrated by the following examples. Examples 1-11 describe the synthesis of suitable intermediates for the compounds of the invention.

**EXAMPLE 1**

A mixture of pyridine-3,4-dicarboxylic acid (15.33 g) and acetic anhydride (30 ml) is refluxed under stirring for one hour. After cooling to room temperature, the acetic anhydride excess is distilled off under reduced pressure and the obtained residue is taken up into ethyl ether (60 ml). The separated solid is filtered and washed with ethyl ether, to yield pyridine-3,4-dicarboxylic acid anhydride (11.72 g); m.p. 76.5-78.5°C.

**EXAMPLE 2**

Using pyridine-2,3-dicarboxylic acid or pyrazine-2,3-dicarboxylic acid in the procedure described in Example 1, pyridine-2,3-dicarboxylic acid anhydride, m.p. 134-136°C, and pyrazine-2,3-dicarboxylic acid anhydride, m.p. 190°C (dec.) are prepared.

**EXAMPLE 3**

A mixture of pyridine-3,4-dicarboxylic acid anhydride (2.43 g) and aluminium chloride (8.60 g) in 1,4-difluorobenzene (32 ml), under vigorous stirring, is heated for 12 hours in oil-bath, kept at 110°C. After recovering the 1,4-difluorobenzene excess by distillation, the obtained residue is cooled in ice-bath and carefully treated with ice-water (35 ml) and concentrated hydrochloric acid (3.6 ml). The obtained precipitate is separated by filtration and washed with water to gave a mixture of 4-(2',5'-difluorobenzoyl)piridine-3-carboxylic acid and 3-(2',5'-difluorobenzoyl)piridine-4-carboxylic

acid (3.02 g, m.p. 217.5-222°C). Said acid mixture (3.0 g), without any further purifications, is added to 20% $SO_3$ oleum (7.5 ml) and the obtained mixture is heated for 2 hrs in oil-bath, kept at 135-140°C. After cooling to room temperature, the mixture is carefully poured into ice (220 ml), neutralized with sodium bicarbonate and extracted with methylene chloride (3 x 60 ml). The combined organic phases are washed with brine and dried over sodium sulfate, then solvent is removed under reduced pressure, to give 6,9-difluorobenzo[g]isoquinoline-5,10-dione in form of a yellow solid; m.p. 199-200°C (2.23 g).
$^1$H-NMR ($CDCl_3$)$\delta$ : 7.57, m, 2H; 8.03, d 1H; 9.12, d, 1H; 9.54, s, 1H.

**EXAMPLE 4**

A mixture of pyridine-2,3-dicarboxylic acid anhydride (2.4 g), aluminium chloride (8.4 g) and 1,4-difluorobenzene (35 ml) is reacted according to the procedure described in Example 3, to give a mixture of 3-(2',5'-difluorobenzoyl)pyridine-2-carboxylic acid and 2-(2',5'-difluorobenzoyl)pyridine-3-carboxylic acid (4.1 g).

Without further purifications, said acid mixture (0.40 g) in 30% $SO_3$ oleum (1.0 ml) is heated for 6 hrs in oil-bath at 155-165°C. The obtained mixture is subsequently treated as described in Example 3, to give 6,9-difluorobenzo[g]quinoline-5,10-dione in form of a yellow solid, m.p. 251-252°C (0.102 g).

**EXAMPLE 5**

A mixture of pyrazine-2,3-dicarboxylic acid anhydride (1.0 g), aluminium chloride (2.3 g) and 1,4-difluorobenzene (22 ml) is reacted according to the procedure described in Example 3, to give crude 3-(2',5'-difluorobenzoyl)-2-pyrazinecarboxylic acid, which is purified by dissolution in chloroform (85 ml), filtration of the insoluble material and extraction of the chloroform solution with a sodium bicarbonate saturated solution. The combined extracts are acidified with 10% sulfuric acid and extracted with chloroform, to give, after distillation of the solvent, said acid (1.24 g), m.p. 149-150°C. This compound is then treated (0.28 g) with 30% $SO_3$ oleum (1.5 ml) at 140°C for 6 hrs, as described in Example 3, to give 6,9-difluorobenzo[g]quinoxaline-5,10-dione in form of a yellow solid, m.p. 230°C (dec.) (0.19 g).

**EXAMPLE 6**

A finely ground mixture of pyridine-3,4-dicarboxylic acid anhydride (4.81 g) and 1,4-dimethoxybenzene (3.08 g) is added in portions in about 3 min to a melted mixture of aluminum chloride (60 g) and sodium chloride (12 g) at 180°C. At the end of the addition the mixture is kept at this temperature for 5 more min, then, while it is still hot and fluid, it is carefully poured into a 0.7N sulfuric acid solution, cooling with an ice-bath. Subsequently the mixture is continuously extracted with chloroform, the chloroform extract is dried, solvent is removed under reduced pressure and the residue is recrystallized from ethanol, to give 6,9-dihydroxybenzo[g]isoquinoline-5,10-dione (1.08 g), m.p. 205-207°C.

**EXAMPLE 7**

Following the procedure described in Example 6 and using pyridine-2,3-dicarboxylic acid anhydride as the starting material, 6,9-dihydroxybenzo[g]quinoline-5,10-dione, m.p. 234-237°C, is prepared.

**EXAMPLE 8**

A solution of 6,9-dihydroxybenzo[g]isoquinoline-5,10-dione (0.25 g) and p-toluenesulfonyl chloride (0.79 g) in pyridine (10 ml) is stirred at room temperature for 48 hrs. After removing pyridine under reduced pressure, the obtained residue is added with water and the separated solid is filtered and washed with cool ethyl acetate. After recrystallization from a chloroform/ligroin mixture, 6,9-bis(p-toluenesulfonyloxy)benzo[g]isoquinoline-5,10-dione (0.35 g), m.p. 201-202°C, is obtained.

**EXAMPLE 9**

Following the procedure described in Example 8 and using 6,9-dihydroxybenzo[g]quinoline-5,10-dione as the starting material, 6,9-bis(p-toluenesulfonyloxy)benzo[g]quinoline-5,10-dione is prepared.

**EXAMPLE 10**

A solution of 6,9-difluorobenzo[g]isoquinoline-5,10-dione (0.97 g) in methylene chloride (40 ml) is added in portions to m-chloroperbenzoic acid (1.78 g, titre about 50%). After about 5 hrs at room temperature, more m-chloroperbenzoic

acid (0.35 g) is added and the resulting mixture is left at room temperature for overall 22 hrs. The obtained solution is washed with a sodium bicarbonate saturated solution (3 x 20 ml), the combined aqueous phases are re-extracted with methylene chloride (20 ml) and the combined organic phases are finally washed with brine. After drying over sodium sulfate and distilling the solvent under reduced pressure, the obtained residue is recrystallized from methylene chloride, to gave 6,9-difluorobenzo[g]isoquinoline-5,10-dione-2-oxide, in form of a yellow solid, m.p. 250.1-252.3°C (0,75 g). $^1$H-NMR ($CDCl_3$)$\delta$ : 7.58, m, 2H; 8.11, d, 1H; 8.43, dd, 1H; 8.82, dd, 1H.

**EXAMPLE 11**

Following similar procedures to those described in Example 10, subjecting the appropriate difluoro- or bis(p-toluenesulfonyloxy)-derivatives of aza- and diaza-anthracenediones to oxidation with suitable oxidating agents, the following compounds are prepared:

6,9-difluorobenzo[g]quinoline-5,10-dione-1-oxide;
6,9-bis(p-toluenesulfonyloxy)benzo[g]quinoline-5,10-dione-1-oxide;
6,9-bis(p-toluenesulfonyloxy)benzo[g]isoquinoline-5,10-dione-2-oxide;

**EXAMPLE 12**

N,N-Dimethylethylenediamine (0.33 ml) is added to a suspension of 6,9-difluorobenzo[g]isoquinoline-5,10-dione-2-oxide (0.20 g) in anhydrous pyridine (5 ml) kept under stirring at 67°C. The reaction mixture is kept at this temperature for 5 hrs, then it is left at room temperature overnight. The obtained solution is applied on a silica gel chromatography column, which is eluted first with ethyl acetate, then with 4/1 v/v ethyl acetate/methanol, finally with 16/4/0.2 v/v/v chloroform/ methanol/conc. ammonia. The fractions from the main blue chromatographic band are combined, concentrated to dryness and the residue is taken up into ethyl ether (10 ml), under stirring, to gave 6,9-bis[N-(2-dimethylaminoethyl)amion]benzo[g]isoquinoline-5,10-dione-2-oxide (0.15 g), m.p. 178°C.
$^1$H-NMR ($CDCl_3$) $\delta$ : 2.40, s, 12H; 2.70, t, 4H; 3.55,q, 4H; 7.35, s, 2H; 8.17, d, 1H; 8.35, dd, 1H; 9.01, d, 1H; 11.10, m, 2H (exchangeable with $D_2O$).
U.V. ($H_2O$) $\lambda_{max}$ (nm) $E_{1\%}$ (cm): 278 (653); 368 (112); 621 (317); 661 (343).
H.P.L.C.: Lichrospher® 100 RP18 (5 $\mu$m); eluent 10 mM sodium heptanesulfonate + 10 mM $KH_2PO_4$ in 70/20/10 water/acetonitrile/dioxane, pH ~ 3 with $H_3PO_4$, flow 1 ml/min. R.t. = 2.85 min.

**EXAMPLE 13**

Following the procedure described in Example 12, by reacting 6,9-difluorobenzo[g]isoquinoline-5,10-dione-2-oxide with 1,2-ethylenediamine, 6,9-bis[N-(2-aminoethyl)amino]benzo[g]isoquinoline-5,10-dione-2- oxide, m.p. 164°C (dec.) is prepared. A solution of this compound (0.35 g) in 1/1 v/v chloroform/methanol (10 ml), kept under stirring in an oil-bath at 60°C, is added with a solution of maleic acid (0.25 g) in methanol (1.5 ml). The reaction mixture is kept at this temperature for 5 min, then it is allowed to cool to room temperature. After two hrs, the separated solid is filtered, washed with methanol (0.5 ml) and ethyl ether and subsequently recrystallized at 70-80°C by dissolution in water (4 ml) and reprecipitation with absolute ethanol (20 ml) to give 6,9-bis[N-(2-aminoethyl)amino]benzo[g]isoquinoline-5,10-dione-2-oxide dimaleate 1.5 $H_2O$, m.p. 183.5°C (dec.) (0.36 g).
$^1$H-NMR ($D_2O$) $\delta$ : 3.35, m, 4H; 3.85, m, 4H; 6.20, s, 4H; 7.35, s, 2H; 7.97, d, 1H; 8.40, dd, 1H; 8.65, d, 1H.
U.V. ($H_2O$): $\lambda_{max}$ (nm), $E_{1\%}$ (cm): 212 (703); 275 (520); 614 (251); 658 (272).
H.P.L.C.: Lichrospher® 100 RP18 (5 $\mu$m); eluent: 10 mM sodium heptanesulfonate + 10 mM $KH_2PO_4$ in 70/20/10 water/acetonitrile/dioxane, pH ~ 3 with $H_3PO_4$; flow 1 ml/min.; R.t. = 2.74 min.

**EXAMPLE 14**

A 6.9N HCl solution in methanol (0.22 ml) at 0°C under magnetic stirring, is dropped into a 6,9-bis[N-(2-dimethylaminoethyl)amino]benzo[g]isoquinoline-5,10-dione-2-oxide (0.19 g) solution in anhydrous chloroform (30 ml). After one hour the obtained suspension is diluted with ethyl ether (30 ml) and the separated blue solid is filtered under nitrogen and washed with ethyl ether, to give 6,9-bis[N-(2-dimethylaminoethyl)amino]benzo[g]isoquinoline-5,10-dione-2-oxide dihydrochloride (0.16 g).
$^1$H-NMR ($D_2O$) $\delta$ : 2.60, m, 12H; 2.90, m, 4H; 3.85, m, 4H; 7.34, s, 2H; 7.95, d, 1H; 8.38, dd, 1H; 8.65, d, 1H.

**EXAMPLE 15**

Following the procedures described in Example 12, by reacting the appropriate nitrogen oxides, prepared in Examples 10 and 11, with the suitable amines, the following compounds are prepared, which are isolated as free bases:

6,9-bis[N-(2-diethylaminoethyl)amino]benzo[g]isoquinoline-5,10-dione-2-oxide
$^1$H-NMR ($CDCl_3$) $\delta$ : 1.15, t, 12H; 2.63, q, 8H; 2.75, t, 4H; 3.58, q, 4H; 7.36, s, 2H; 8.15, d, 1H; 8.35, dd, 1H; 9.03, d, 1H; 11.15, m, 2H.
6,9-bis{N-[2-(1-pyrrolidinyl)ethyl]amino}benzo[g]isoquinoline-5,10-dione-2-oxide
$^1$H-NMR ($CDCl_3$) $\delta$ : 1.87, m, 8H; 2.69, m, 8H; 2.85, m, 4H; 3.62, m, 4H; 7.38, s, 2H; 8.17, d, 1H; 8.37, dd, 1H; 9.03, d, 1H; 11.00, t, 1H; 11.11, t, 1H.
6,9-bis{N-[2-(4-morpholinyl)ethyl]amino}benzo[g]isoquinoline-5,10-dione-2-oxide
$^1$H-NMR ($CDCl_3$) $\delta$ : 2.55, m, 8H; 2.80, m, 4H; 3.59, m, 4H; 3.78, m, 8H; 7.35, s, 2H; 8.15, d, 1H; 8.36, dd, 1H; 9.01, d, 1H; 10.95-11.03, brt, 2H.
6,9-bis{N-[2-(2-hydroxyethylamino)ethyl]amino}benzo[g]isoquinoline-5,10-dione-2-oxide
$^1$H-NMR ($CDCl_3$) $\delta$ : 2.98, m, 4H; 3.10, m, 4H; 3.55, m, 4H; 3.86, m, 4H; 7.35, s, 2H; 8.15, d, 1H; 8.38, dd, 1H; 9.02, d, 1H; 11.15, m, 2H.
6,9-bis{N-[2-(1-aziridinyl)ethyl]amino}benzo[g]isoquinoline-5,10-dione-2-oxide
$^1$H-NMR ($CDCl_3$) $\delta$ : 1.65, m, 8H; 2.65, m, 4H; 3.60, m, 4H; 7.34, s, 2H; 8.17, d, 1H; 8.35, dd, 1H; 9.01, d, 1H; 11.11, m, 2H.
6,9-bis{N-(3-hydroxypropyl)amino]benzo[g]isoquinoline-5,10-dione-2-oxide
$^1$H-NMR ($CDCl_3$) $\delta$: 2.11, m, 4H; 3.58,m, 4H; 3.65, m, 4H; 7.36, s, 2H; 8.15, d, 1H; 8.38, dd, 1H; 9.03, d, 1H; 11.13, m, 2H.
6,9-bis[N-(2-hydroxyethyl)amino]benzo[g]isoquinoline-5,10-dione-2-oxide
$^1$H-NMR ($CDCl_3$) $\delta$ : 3.60, m, 4H; 3.69, m, 4H; 7.34, s, 2H; 8.14, d, 1H; 8.37, dd, 1H; 9.01, d, 1H; 11.15, m, 2H.
6,9-bis{N-(1,1-bis(hydroxymethyl)methyl)amino]benzo[g]isoquinoline-5,10-dione-2-oxide
$^1$H-NMR($CDCl_3$) $\delta$ : 3.80-3.92, m, 10H; 7.34, s, 2H; 8.15, d, 1H; 8.36, dd, 1H; 9.01, d, 1H; 11.13; m, 2H.
6,9-bis[N-(3-aminopropyl)amino]benzo[g]isoquinoline-5,10-dione-2-oxide;
6,9-bis{N-[2-(methylcarbamoylamino)ethyl]amino}benzo[g]isoquinoline-5,10-dione-2-oxide;
6,9-bis{N-[2-(phenylcarbamoylamino)ethyl]amino}benzo[g]isoquinoline-5,10-dione-2-oxide;
6,9-bis[N-(2-dimethylaminoethyl)amino]benzo[g]quinoline-5,10-dione-1-oxide;
6,9-bis[N-(3-aminopropyl)amino]benzo[g]quinoline-5,10-dione-1-oxide;
6,9-bis{N-[2-(2-hydroxyethylamino)ethyl]amino}benzo[g]quinoline-5,10-dione-1-oxide;
6,9-bis[N-(2-aminoethyl)amino]benzo[g]-quinoline-5,10-dione-1-oxide.

**EXAMPLE 16**

By subjecting the appropriate free bases to the salification processes described in Examples 13 and 14, the following maleate or hydrochloride salts are prepared:

6,9-bis[N-(3-aminopropyl)amino]benzo[g]isoquinoline-5,10-dione-2-oxide dimaleate
$^1$H-NMR ($D_2O$) $\delta$ : 2.30, m, 4H; 3.37, m, 4H; 3.86, m, 4H; 6.23, s, 4H; 7.36, s, 2H; 7.97, d, 1H; 8.40, dd, 1H; 8.67, d, 1H.
6,9-bis[N-(4-aminobutyl)amino]benzo[g]isoquinoline-5,10-dione-2-oxide dihydrochloride
$^1$H-NMR ($D_2O$) $\delta$ : 2.18, m, 8H; 3.35, m, 4H; 3.84, m, 4H; 7.33, s, 2H; 7.95, d, 1H; 8.38, dd, 1H; 8.65, d, 1H.
6,9-bis{N-[2-(4-morpholinyl)ethyl]amino}benzo[g]isoquinoline-5,10-dione-2-oxide dihydrochloride;
6,9-bis[N-(3-aminopropyl)amino]benzo[g]quinoline-5,10-dione-1-oxide dimaleate.
6,9-bis[N-(2-aminoethyl)amino]benzo[g]quinoline-5,10-dione-1-oxide dimaleate.

## Claims

1. Compounds of formula (I):

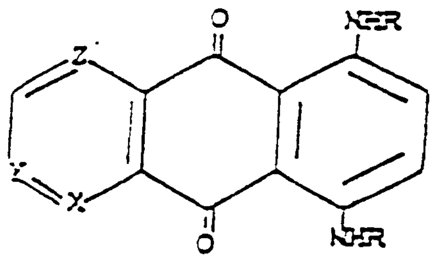

(I)

wherein

X, Y and Z are CH, N or N → O thus forming an azine or diazine ring, with the proviso that:

- at least one of X, Y and Z is N → O;

R is a substituted ($C_2$-$C_{10}$ )-alkyl group, selected from the group consisting of :

- residues of formula $-(CH_2)_p$-OH, wherein p is an integer from 2 to 4;
- residues of formula $-(CH_2)_p-NH_2$ wherein p is as defined above;
- residues of formula $-(CH_2)_p-NR^2R^3$ wherein p is as defined above and $R^2$ and $R^3$ are ($C_1$-$C_6$)-alkyl groups or, taken together with the nitrogen atom, they form an aziridine, pyrrolidine, morpholine, piperazine, N-methylpiperazine, N-benzylpiperazine or piperidine ring;
- residues of formula

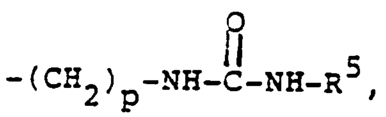

  wherein p is as defined above and $R^5$ is ($C_1$-$C_6$)-alkyl or phenyl;
- residues of formula $-(CH_2)_p-NH-(CH_2)_q$-OH wherein p and q are independently an integer 2 to 4;
- residues of formula

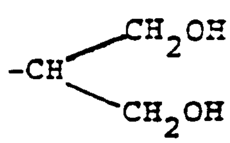

or

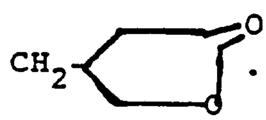

and the pharmaceutically acceptable salts thereof.

**2.** Compounds as claimed in claim 1 wherein X and Z are CH and Y is N → O.

**3.** A compound of claim 1 which is 6,9-bis[N-(2-dimethylaminoethyl)amino]benzo[g]isoquinoline-5,10-dione-2-oxide.

**4.** A compound of claim 1 which is 6,9-bis[N-(2-aminoethyl)amino]benzo[g]isoquinoline-5,10-dione-2-oxide dimaleate.

**5.** A process of the preparation of compounds of claim 1, which comprises reacting a compound of formula (II):

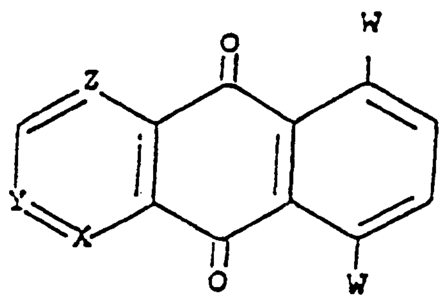

(II)

wherein

X, Y and Z are as defined above;

W is a fluorine atom or a

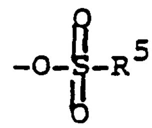

group, $R^5$ being as defined above;

with a compound of formula (III)

$$H_2N\text{-}R_a \qquad \text{(III)}$$

wherein $R_a$ has the same meanings as defined in formula (I) for R or it is a group which may be converted to R, to give a compound of formula (Ia):

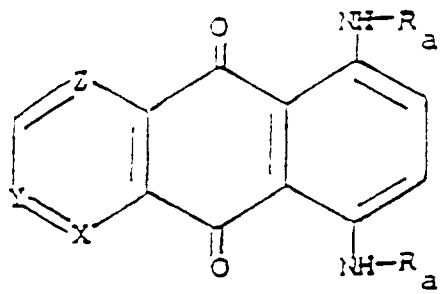

(Ia)

and then optionally performing one or more of the following steps:

a) when $R_a$ is other than R, converting $R_a$ into R to obtain a compound of formula (I);
b) when $R_a$ is one of the groups defined above for R in compounds of formula (I), and in which case the compounds of formula (Ia) are the same as the compounds of formula (I), $R_a$ can be optionally converted into another $R_a$ group to give another compound of formula (I);
c) optional salification and/or solvation of the obtained compounds of formula (I) or separation of the isomers thereof.

**6.** Pharmaceutical compositions containing as the active ingredient a compound as claimed in claims 1- 4, in admixture with appropriate excipients.

7. The use of the compounds as claimed in claims 1- 4 for the preparation of an antitumor medicament.

**Patentansprüche**

1. Verbindungen der Formel (I):

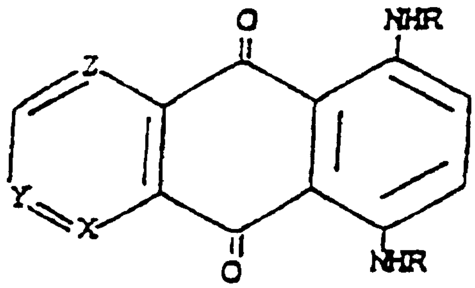

(I)

worin

X, Y und Z CH, N oder N → O sind und so einen Azin- oder Diazin-Ring bilden, mit der Maßgabe, daß:

- wenigstens eines von X, Y und Z N → O ist; R eine substituierte ($C_2$-$C_{10}$)-Alkyl-Gruppe ist, ausgewählt aus der Gruppe bestehend aus:
- Resten der Formel $-(CH_2)_p-OH$, worin p eine ganze Zahl von 2 bis 4 ist;
- Resten der Formel $-(CH_2)_p-NH_2$, worin p wie vorstehend definiert ist;
- Resten der Formel $-(CH_2)_p-NR^2R^3$, worin p wie vorstehend definiert ist und $R^2$ und $R^3$ ($C_1$-$C_6$)-Alkyl-Gruppen sind oder zusammengenommen mit dem Stickstoff-Atom einen Aziridin-, Pyrrolidin-, Morpholin-, Piperazin-, N-Methylpiperazin-, N-Benzylpiperazin- oder Piperidin-Ring bilden;
- Resten der Formel

$$-(CH_2)_p-NH-\overset{\overset{O}{\|}}{C}-NH-R^5 \quad ,$$

worin p wie vorstehend definiert ist und $R^5$ ($C_1$-$C_6$)-Alkyl oder Phenyl ist;
- Resten der Formel $-(CH_2)_p-NH-(CH_2)_q-OH$, worin p und q ganze Zahlen von 2 bis 4 sind;
- Resten der Formel

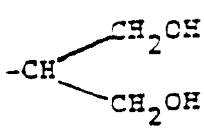

oder

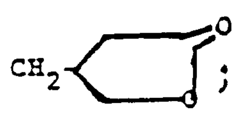

;

und die pharmazeutisch annehmbaren Salze derselben.

2. Verbindungen nach Anspruch 1, worin X und Z CH sind und Y N → O ist.

3. Verbindung nach Anspruch 1, die 6,9-Bis[N-(2-dimethylaminoethyl)amino]benzo[g]isochinolin-5,10-dion-2-oxid ist.

4. Verbindung nach Anspruch 1, die 6,9-Bis[N-(2-aminoethyl)amino]benzo[g]isochinolin-5,10-dion-2-oxid-dimaleat ist.

5. Verfahren zur Herstellung von Verbindungen nach Anspruch 1, umfassend das Umsetzen einer Verbindung der

Formel (II)

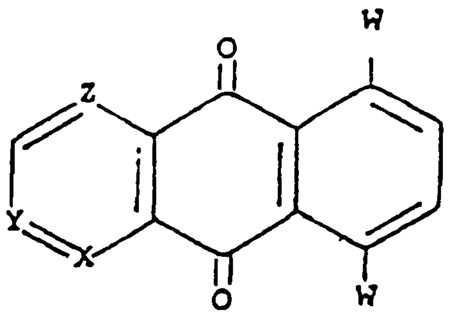

(II)

worin

X, Y und Z wie vorstehend definiert sind;
W ein Fluor-Atom oder eine

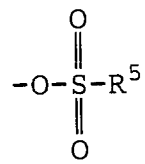

Gruppe ist, wobei $R^5$ wie vorstehend definiert ist;

mit einer Verbindung der Formel (III)

$$H_2N\text{-}R_a \qquad \text{(III)}$$

worin $R_a$ die gleichen Bedeutungen wie in Formel (I) für R definiert hat oder eine Gruppe ist, die sich in R überführen läßt, um eine Verbindung der Formel (Ia)

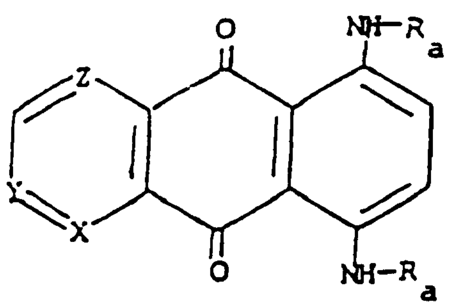

(Ia)

zu ergeben, und dann gegebenenfalls das Durchführen eines oder mehrerer der folgenden Schritte:

a) wenn $R_a$ verschieden von R ist, Überführen von $R_a$ in R, um eine Verbindung der Formel (I) zu ergeben;
b) wenn $R_a$ eine der vorstehend für R definierten Gruppen in den Verbindungen der Formel (I) ist, wobei in diesem Fall die Verbindungen der Formel (Ia) die gleichen wie die Verbindungen der Formel (I) sind, so kann $R_a$ in eine andere $R_a$-Gruppe überführt werden, um eine andere Verbindung der Formel (I) zu ergeben;
c) gegebenenfalls Salzbildung und/oder Solvatation der erhaltenen Verbindung der Formel (I) oder Trennung der Isomere derselben.

**6.** Pharmazeutische Zusammensetzungen, enthaltend als wirksamen Bestandteil eine Verbindung nach den Ansprüchen 1 bis 4 im Gemisch mit geeigneten Trägerstoffen.

**7.** Verwendung der Verbindungen nach den Ansprüchen 1 bis 4 zur Herstellung eines Antitumor-Medikaments.

## Revendications

**1.** Composés de formule (I) :

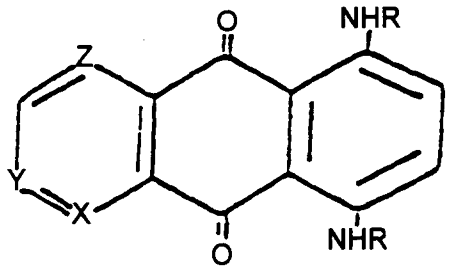

(I)

dans laquelle:

X, Y et Z sont CH, N ou N->O formant ainsi un cycle azine ou diazine, à condition que:

- au moins un de X, Y et Z soit N->O;

R est un groupe alkyle en $C_2$ à $C_{10}$ substitué, choisi dans le groupe formé par:

- les résidus de formule $-(CH_2)_p-OH$, dans laquelle p est un nombre entier de 2 à 4;
- les résidus de formule $-(CH_2)_p-NH_2$, dans laquelle p est tel que défini ci-dessus;
- les résidus de formule $-(CH_2)_p-NR^2R^3$, dans laquelle p est tel que défini ci-dessus et $R^2$ et $R^3$ sont des groupes alkyle en $C_1$ à $C_6$ ou, pris ensemble avec l'atome d'azote, ils forment un cycle aziridine, pyrrolidine, morpholine, pipérazine, N-méthylpipérazine, N-benzylpipérazine ou pipéridine;
- les résidus de formule

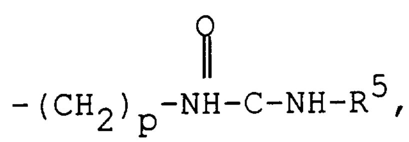

  dans laquelle p est tel que défini ci-dessus et $R^5$ est un groupe alkyle en $C_1$ à $C_6$ ou un groupe phényle;
- les résidus de formule $-(CH_2)_p-NH-(CH_2)_q-OH$, dans laquelle p et q sont indépendamment un nombre entier de 2 à 4;
- les résidus de formule

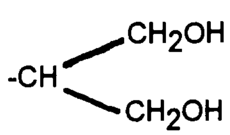

ou

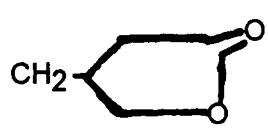

et leurs sels acceptables sur le plan pharmaceutique.

**2.** Composés selon la revendication 1, dans lesquels X et Z sont CH et Y est N->O.

**3.** Composé selon la revendication 1, qui est le 6, 9-bis[N-(2-diméthylaminoéthyl)amino]benzo[ g]isoquinoléine-5,10-dione-2-oxyde.

4. Composé selon la revendication 1, qui est le dimaléate de 6,9-bis[ N-(2-aminoéthyl)amino]benzo[ g]-isoquinoléine-5,10-dione-2-oxyde.

5. Procédé de la préparation de composés selon la revendication 1, qui consiste à faire réagir un composé de formule (II):

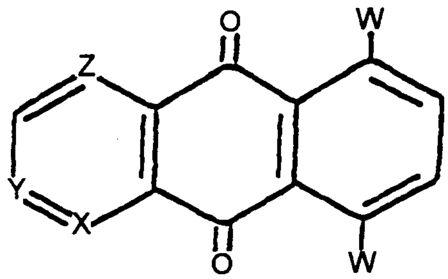

(II)

dans laquelle

X, Y et Z sont tels que définis ci-dessus;

W est un atome de fluor ou un groupe

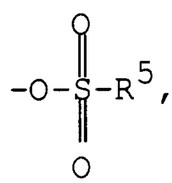

$R^5$ étant tel que défini ci-dessus;

avec un composé de formule (III)

$H_2N\text{-}R_a$ (III)

dans laquelle $R_a$ possède les mêmes significations que définies dans la formule (I) pour R ou c'est un groupe qui peut être converti en R, pour obtenir un composé de formule (Ia) :

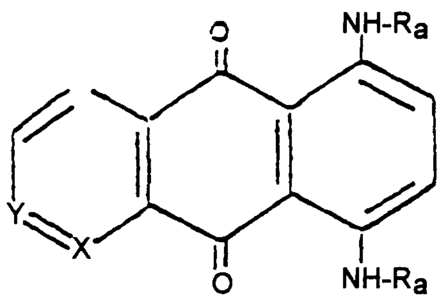

(Ia)

puis éventuellement à réaliser une ou plusieurs des étapes suivantes consistant:

a) quand $R_a$ est autre que R, à convertir $R_a$ en R pour obtenir un composé de formule (I);

b) quand $R_a$ est un des groupes définis ci-dessus pour R dans les composés de formule (I), et auquel cas les composés de formule (Ia) sont les mêmes que les composés de formule (I), à convertir éventuellement $R_a$ en un autre groupe $R_a$ pour obtenir un autre composé de formule (I);

c) à salifier et/ou à solvater éventuellement les composés obtenus de formule (I) ou à séparer leurs isomères.

**6.** Compositions pharmaceutiques contenant en tant que principe actif un composé selon les revendications 1 à 4, en mélange avec des excipients appropriés.

**7.** Utilisation des composés selon les revendications 1 à 4, pour la préparation d'un médicament antitumoral.